(19) Europäisches Patentamt
European Patent Office
Office européen des brevets

(11) Publication number: **0 040 315**
**B1**

(12) # EUROPEAN PATENT SPECIFICATION

(45) Date of publication of patent specification: **24.10.84**

(51) Int. Cl.$^3$: **C 07 C 51/60,** C 08 G 63/64

(21) Application number: **81102683.0**

(22) Date of filing: **09.04.81**

(54) **Interfacial production of poly(ester carbonate) or polyester including acid chloride synthesis.**

(30) Priority: **02.05.80 US 145901**
**02.05.80 US 146942**

(43) Date of publication of application:
**25.11.81 Bulletin 81/47**

(45) Publication of the grant of the patent:
**24.10.84 Bulletin 84/43**

(84) Designated Contracting States:
**DE FR GB IT**

(56) References cited:
**EP-A-0 006 965**
**EP-A-0 013 196**
**EP-A-0 017 740**
**EP-A-0 021 373**
**EP-A-0 024 492**
**US-A-4 129 594**
**US-A-4 156 069**
**US-A-4 194 038**
**US-A-4 219 635**

(73) Proprietor: **ALLIED CORPORATION**
**Columbia Road and Park Avenue P.O. Box 2245R**
**(Law Dept.)**
**Morristown New Jersey 07960 (US)**

(72) Inventor: **Cooke, Robert Sanderson**
**41 Burnham Road**
**Morris Plains New Jersey 07950 (US)**
Inventor: **DeBona, Bruce Todd**
**23 Rose Avenue**
**Madison New Jersey 07940 (US)**
Inventor: **Dege, Gerald Joseph**
**204 Mooney Road**
**Flanders New Jersey 07836 (US)**
Inventor: **Segal, Leon**
**5 Lookout Road**
**Randolph New Jersey 07869 (US)**
Inventor: **Van Buskirk, Bruce**
**100 Center Grove Road Apt. 9-4**
**Randolph New Jersey 07869 (US)**

(74) Representative: **Baillie, Iain Cameron et al**
**c/o Langner Parry Isartorplatz 5**
**D-8000 München 2 (DE)**

**EP 0 040 315 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Background of the Invention

The production of a poly(ester carbonate) from monomers such as bisphenol A, terephthaloyl chloride and phosgene is described in U.S. Patents 4,156,069 of Prevorsek et al. and 4,194,038 of Baker et al. In U.S. Patent 4,194,038 and 4,129,594, the terephthaloyl chloride, or similar acid chloride, is synthesized from the corresponding aromatic or cycloaliphatic acid by reaction with phosgene in organic solution. Thereafter, in U.S. Patent 4,194,038, phosgene and a dihydric aromatic alcohol such as bisphenol A are added in a prescribed manner to the solution to produce the poly(ester carbonate). Accordingly, the process of U.S. Patent 4,194,038 provides for the production of the poly(ester carbonate) from the aromatic or cycloaliphatic dicarboxylic acid without isolation of the acid chloride by solution processes in a single reaction chamber.

For various reasons, it is often desirable commercially to produce condensation polymers such as polyesters and poly(ester carbonates) by an interfacial process in which the aromatic dihydric alcohol is introduced as an aqueous solution of the corresponding alkali metal salt. Such an interfacial process is described in copending, commonly assigned U.S. Patent Application 029,422 of Segel et al., filed April 12, 1979, published October 29, 1980 as European Published Application 17,740 and issued March 10, 1981 as U.S. Patent 4,255,556 proceeding from the acid chloride such as terephthaloyl chloride as the reactant. Terephthaloyl chloride is introduced in relatively pure form, and no consideration is given in the disclosure of this copending application to complications due to the presence of catalysts or by-products from the production of the acid chloride. It would be desirable to produce poly(ester carbonates) by an interfacial process in a single reaction chamber or, at least, to produce same from the aromatic or cycloaliphatic acid chloride without isolation or extensive purification of the acid chloride from catalysts or by-products of its synthesis.

Acid chlorides of dicarboxylic acids are often used as monomers in the production of condensation polymers such as polyesters and poly(ester carbonates). Methods have been described for producing terephthalic and isophthalic acid chlorides, for example, from corresponding trichloromethyl compounds (e.g. hexachloroxylene), from alkyl esters (e.g. dimethyl terephthalate) and especially from the free acids (e.g. terephthalic acid).

One method of producing acid chlorides is by the use of phosgene to chlorinate the free acid. Thus, for terephthalic or isophthalic acid chloride, the following reaction has been used:

$$HOOC—C_6H_4—COOH + 2COCl_2 \rightarrow ClOC—C_6H_4—COCl + 2CO_2 + 2HCl$$

In general, the reaction has required a tertiary nitrogen catalyst in and temperature sufficiently elevated for the by-product HCl to be removed as a gas.

A process is described in U.S. Patent 4,129,594 (December 12, 1978 to Baker et al.) to conduct the above reaction at moderate temperatures in an inert chlorinated paraffinic hydrocarbon solvent with sufficient organic base (e.g. pyridine) present to neutralize the HCl as formed. This process produces the acid chloride in high yields without forming substantial amounts of anhydride. The disadvantage is the presence of large amounts of neutralized HCl (e.g. pyridine hydrochloride, also called pyridinium chloride) in the organic solution with the acid chloride. Pyridinium chloride is acceptable when the acid chloride is to be further reacted in a solution condensation process wherein additional HCl is to be neutralized, as in U.S. Patent 4,194,038 (March 13, 1980 to Baker et al.). The presence of a neutralized HCl is undesirable, however, if the acid chloride is to be purified or if it is to be used in an interfacial process.

Brief Description of the Invention

It has been discovered that aromatic and cycloaliphatic dicarboxylic acid chlorides can be prepared from phosgene and an alakali metal or alkaline earth metal salt of an aromatic or cycloaliphatic dicarboxylic acid using a catalystic amount of an organic base.

Accordingly, the present invention includes in its first form a method of preparing an aromatic or cycloaliphatic dicarboxylic acid chloride which comprises reacting an alkali metal or alkaline earth metal salt of an aromatic or cycloaliphatic dicarboxylic acid in an inert organic solvent with phosgene in the presence of at least one tertiary nitrogen compound with a pyridine nucleus in a molar amount of 0.1% to 25% by moles of carboxylate, to form the corresponding aromatic or cycloaliphatic dicarboxylic acid chloride dissolved in the organic solvent, carbon dioxide and a solid alkali metal or alkaline earth metal chloride salt.

It has been discovered that such processes proceed with reasonably rapid rates, without the formation of substantial amounts of the anhydride, yielding the acid chloride in organic solution with relatively low amounts of organic base present.

It has also been discovered that aromatic and cycloaliphatic acid chlorides can be used in an interfacial polymerization without isolation or extensive purification only if any tertiary nitrogen compound with a pyridine nucleus present in the acid chloride is reduced below a critical level. It has also been discovered that such aromatic and cycloaliphatic acid chlorides can be used directly in interfacial

polymerizations without isolation or extensive purification only by substantial modification of the method by which they are produced from the corresponding acid and phosgene compared to the previously disclosed method of U.S. Patent 4,129,594. The present synthesis process in certain forms (with a particular range of levels of tertiary nitrogen compound) can therefore be combined with an interfacial polymerization to form a poly(ester carbonate).

Accordingly, the present invention also includes, in its second form, a process for the production of a poly(ester carbonte) which comprises:

a) forming an aromatic or cycloaliphatic dicarboxylic acid chloride dissolved in the organic solvent, carbon dioxide and an alkali metal or alkaline earth metal chloride salt suspended in the organic solution by reacting an alkali metal or alkaline earth metal salt of an aromatic or cycloaliphatic dicarboxylic acid in an inert organic solvent with phosgene in the presence of at least one tertiary nitrogen compound with a pyridine nucleus in a molar amount of 0.1% to 25% by moles of carboxylate;

b) preferably, but not necessarily, separating the suspended alkali metal or alkaline earth metal chloride from the organic solution; and

c) mixing the organic solution with an aqueous solution of an alkali metal or alkaline earth metal salt of a dihydric aromatic alcohol under sufficient agitation and with sufficient phosgene and tertiary nitrogen polymerization catalyst present to form a poly(ester carbonate) polymer.

The present invention also includes a process for the production of a polyester which comprises:

a) forming an aromatic or cycloaliphatic dicarboxylic acid chloride dissolved in the organic solvent, carbon dioxide and an alkali metal or alkaline earth metal chloride salt suspended in the organic solution by the method of the first form of the invention,

b) preferably, but not necessarily, separating the suspended alkali mental or alkaline earth metal chloride from the organic solution; and

c) removing any unreacted phosgene from the organic solution; and

d) mixing the organic solution with an aqueous solution of an alkali metal or alkaline earth metal salt of a dihydric aromatic alcohol under sufficient agitation and with sufficient tertiary nitrogen polymerization catalyst present to form a polyester polymer.

Detailed Description of the Invention

The present invention involves, in at least one form, the reaction of an alkali metal or alkaline earth metal salt of an aromatic or cycloaliphatic dicarboxylic acid with phosgene in an inert organic solvent in the presence of a tertiary nitrogen compound preferably containing a pyridine nucleus to form the corresponding aromatic or cycloaliphatic dicarboxylic acid chloride. Suitable aromatic dicarboxylic acids include substituted and unsubstituted forms of isophthalic and terephthalic acid, naphthalenedicarboxylic acid, benzophenonedicarboxylic acid, anthracenedicarboxylic acid and similar acids represented generally by the following formulae:

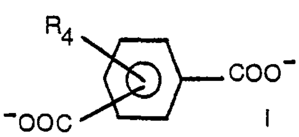
I

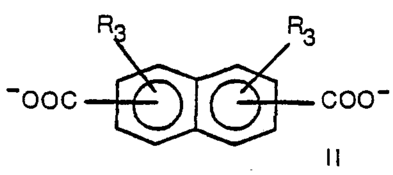
II

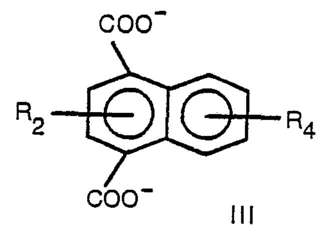
III

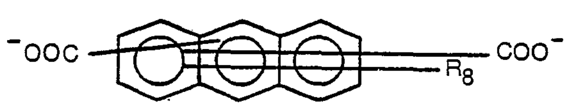
IV

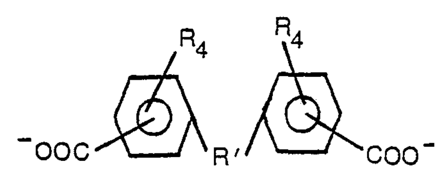
V

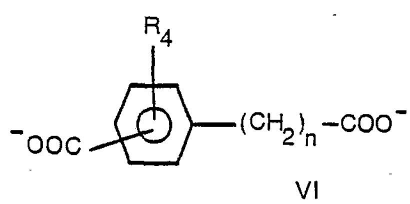
VI

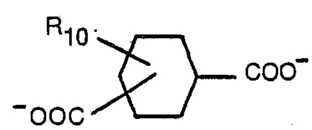
VII

Thus, suitable carboxylate portions of this salt include terephthalate, isophthalate and substituted forms thereof as represented by the above formula I. Also suitable are dicarboxylates of fused ring

compounds such as represented by above formulas II, III and IV. Also suitable are dicarboxylates of multi-ring aromatic moieties which are not fused such as indicated by above formula V. It is also suitable to use dicarboxylates wherein one or both of the carboxyls are not directly linked to the aromatic nucleus such as, for example, those shown in formula VI wherein n is 1 to 5.

Also suitable are cycloaliphatic dicarboxylates such as of above formula VII.

In each of the above formulae, it is preferred that the carboxylates not be on adjacent carbons or in other arrangements (e.g. 1,8-substitution in formula II) where there is a tendency for rapid intramolecular anhydride formation, as with the formation of phthalic anhydride rather than phthalic acid chloride. It will be appreciated that rings of different sizes and other possible arrangements of carboxylates are possible.

In each of the above formulae, substituents designated R may be H, Cl, Br, F, alkyl, alkoxy, phenyl or any other non-reactive substituent. Preferably R is H in all occurrences. In formula V, the linking substituent R′ may be —O—, —S—, —$SO_2$—, alkylene (such as isopropylidene), —CO—, a single bond or any other inert divalent radical.

The alkali or alkaline earth metal which forms a part of the above starting material may be alkali metal, such as lithium, sodium or potassium, and may also be alkaline earth such as magnesium or calcium. In general, the alkali metal salts are preferred, sodium and potassium salts are more preferred and sodium salts are most preferred.

The preferred group of alkali metal or alkaline earth metal salts of aromatic dicarboxylic acids are the disodium and dipotassium salts of terephthalic or isophthalic acids. Most preferred is the disodium salt of terephthalic acid.

The method of the present invention is conducted in an inert organic solvent. The term inert is used to mean that the solvent does not interfere with the reaction, nor does it consume any of the reactants. It is not intended, however, that the solvent play no role in the reaction. Furthermore, the term solvent is not meant to require that each reactant, product and by-product dissolve in the solvent. In fact, it is preferred that only the product acid chloride, the reactant phosgene and the tertiary nitrogen compound be soluble to a significant degree in the solvent. It is not critical whether or not the reactant alkali metal or alkaline earth metal salt of an aromatic or cycloaliphatic dicarboxylic acid is soluble to a significant degree in the solvent and, in the examples, this solubility is quite limited. It is preferred that the by-product inorganic salt (e.g. sodium chloride) be insoluble in the solvent for the system.

The amount of solvent is not critical for the present invention, but it is desirable that sufficient solvent be present to dissolve all of the reactant phosgene, tertiary nitrogen compound and product acid chloride. Excesses of solvent beyond this amount are of no advantage.

In general, the amount of phosgene used in the reaction is not critical, but it is desirable that at least about an equal molar amount of phosgene by moles of carboxylate be provided for complete conversion to the acid chloride. If the system is not carefully kept anhydrous, some allowances may be required to be made for hydrolysis of either the phosgene or the product acid chloride, with the result that some additional amounts of phosgene may be required. It is preferred, however, to use an excess over the stoichiometric amount of phosgene and to use anhydrous reagents and solvent, to increase the reaction rate and reduce the amount of undesired anhydride by-product. Preferably, at least a 10% excess of phosgene is used, such that phosgene is present at least 110%, by moles of a carboxylate. It is more preferred to use at least a 50% excess, such that phosgene is present at least 150%, by moles of carboxylate. It will be appreciated that a 10% excess by moles of carboxylate will result in an organic solution with about 5 moles of diacid chloride per mole of unreacted phosgene. If a poly(ester carbonate) is to be produced, then it is more preferred to use from a 20% excess to an amount of phosgene sufficient for both diacid chloride production and formation of carbonate linkages. Thus, if the acid chloride is to be used to produce a poly(ester carbonate) of about equal parts carbonate and aromatic diacid-derived moieties, then 120% to 150% of phosgene by moles of carboxylate is the more preferred range.

The order of addition of reactants is not critical, but it is preferred that the phosgene be present in the reaction mixture at least as early as the reactant carboxylate salt. Thus, it is preferred to add the carboxylate salt to the solvent already contained the phosgene rather than the reverse. In general, if the carboxylate is added to a solution which contains acid chloride, but which does not contain phosgene, then the somewhat slower reaction between the carboxylate salt and the product acid chloride to form anhydride will occur. When phosgene is present, however, it is believed that the chlorination reaction successfully competes with the anhydride forming reaction with the result that little anhydride is formed.

The reaction is conducted in the presence of a catalytic (i.e. less than stoichiometric by moles of carboxylate) amount of a tertiary nitrogen compound. The requirements for the tertiary nitrogen compound are that it is soluble in the solvent, at least to a limited degree. Tertiary nitrogen compounds having three separate organic substituents may be used. However, it is preferred to use tertiary nitrogen compounds having the nitrogen as part of a heterocyclic ring such as in a quinoline or pyridine nucleus, with compounds having a pyridine nucleus being more preferred. Most preferred is pyridine itself.

While the quantity of tertiary nitrogen compound present is not critical, the advantages of the

present invention are best achieved using substantially less tertiary nitrogen compound, by moles, than the moles of carboxylate present. Preferably, the tertiary nitrogen compound, at least in the case of those with the pyridine nucleus, is present in a molar amount of 0.1 to 25%, by moles of carboxylate. For the processes of producing polyesters and poly(ester carbonates), about 10% is the preferred upper limit. More preferably, the compound with the pyridine nucleus is present in a molar amount of 0.2 to 5%, by moles of carboxylate, with 0.5 to 3% being most preferred.

In general, the greater the amount of tertiary nitrogen compound present, the faster the reaction rate. It has been determined, however, that the reaction rate is sufficiently fast, even with the small preferred amounts of tertiary nitrogen compound, to be acceptable. It should be appreciated that, because the present reaction produces carbon dioxide as a by-product, in actual operations the rate may be limited by the ability to withdraw the carbon dioxide and strip it of entrained phosgene and solvent. Thus, even with low amounts of tertiary nitrogen compound present, it may be possible to achieve the maximum reaction rate for the carbon dioxide removal equipment. Since the reaction rate is a function of the particular reactants and tertiary nitrogen compound, the concentration of tertiary nitrogen compound, the particular solvent, the concentration of all ingredients in the solvent and the temperature at which the reaction is conducted, it is difficult to generalize on the reaction rate. Nevertheless, no more than routine experimentation is required to determine the desirable concentrations of the various reactants and tertiary nitrogen compound for a particular reaction in a particular solvent.

The pressure of the reaction is not critical, but the use of atmospheric pressure as in the Examples limits the usable reaction temperature to less than the boiling point of the solvent (about 40°C for dichloromethane). With superatmospheric pressures, higher temperature can be used without solvent loss. Assuming that reaction rates continue to rise as they do from 25°C to 35°C (see Table I, below), smaller amounts of tertiary nitrogen compound should give equivalent reaction rates at higher pressures. Thus, even less than 0.1 or 0.5% tertiary nitrogen compound by moles of carboxylate may be suitable at superatmospheric pressures and elevated temperatures.

Once the acid chloride is synthesized by such process, it is desirable to remove the by-product alkali metal or alkaline earth metal chloride salt before proceeding with polymerization. Since this inorganic salt is generally insoluble in the organic phase, separation may be conducted by centrifugation, filtration or other conventional means. The separation, while desirable, is not required in view of subsequent addition of the aqueous bisphenolate phase in which the inorganic salt would dissolve. It may be desirable, however, to remove the inorganic salt before introducing the aqueous bisphenolate in that the inorganic salt concentration may otherwise become excessive during polymerization. Any by-product dicarboxylic acid or anhydride present would also be found preferentially in the solid phase.

Removing the inorganic salt has the additional advantages that the reaction mixture may become easier to stir and easier to separate from a non-miscible phase. Normally other undesirable materials such as by-product anhydride and unreacted carboxylic acid salt are removed with the inorganic salt. If the subsequent reaction is to produce a poly(ester carbonate), then the excess phosgene is normally left in solution and the solvent amount is either left unchanged or increased. If the subsequent reaction is to produce polyester, then any excess phosgene should be removed as by evaporating off, evaporating with it some solvent if the solvent is volatile. Additional solvent may then be added, with the desirable solvent level for either polymerization being that sufficient to keep polymer as formed in solution without excessive viscosities developing that hinder good mixing of phases during reaction or hinder good separation of phases after reaction or hinder handling.

One form of the present invention employs a cycloaliphatic or aromatic dicarboxylic acid chloride in an organic solvent having an amount of tertiary nitrogen compound having a pyridine nucleus between 0.1 and 10 percent by moles of carbonyl halide (preferably 0.2—5 percent) for interfacial reaction with an aqueous bisphenolate solution. While the organic phase is preferably prepared by a process as described above, it may also be formed by other means. The interfacial polymerization reaction is conducted by agitating the organic phase with an aqueous bisphenolate phase as described below.

The aqueous bisphenolate phase contains an alkali metal salt or alkaline earth metal salt of a dihydric aromatic alcohol such as the sodium salt of bisphenol A. It is permissible, however, to use any bisphenol such as hydroquinone, phenolphthalein and o,o,o′,o′-tetramethyldicumylbisphenol as in copending, commonly assigned U.S. Patent Applications 133,227 (March 24, 1980), published May 6, 1981 as European Published Application 27,844, and 133,228 (March 24, 1980) of Prevorsek and DeBona, issued January 12, 1982 as U.S. Patent 4,310,652.

If some phosgene is left in the organic phase, then poly(ester carbonate) polymers result with, depending on the phosgene to acid chloride ratio, anywhere from 5 to 60 parts carbonate residues and from 40 to 95 parts diacyl residues for each 100 parts bisphenol residues.

If the phosgene is removed before polymerization, or never present as an excess over that required for acid chloride synthesis, then the product will be a polyester with about equal parts diacyl and bisphenol residues.

During the polymerization, it is desirable that a basic catalyst for polymerization be present together, in the case of poly(ester carbonate) synthesis, with sufficient phosgene to constitute at least 20 percent of the stoichiometric amount for completion of the polymerization reaction. The basic polymerization catalyst may be tertiary nitrogen compound present from the acid chloride synthesis. It is highly preferred, however, to use a tertiary nitrogen compound with a pyridine nucleus such as pyridine itself, for the acid chloride synthesis, and then use a tertiary nitrogen compound with three separate substituents such as a trialkylamine (e.g. triethylamine) for the polymerization. The same compound, e.g. 4-dimethylaminopyridine, may contain both functionalities. In the preferred case, however, the acid chloride catalyst (e.g. pyridine) is different from the polymerization catalyst (e.g. triethylamine). The phosgene may be carried over from the synthesis of acid chloride, and preferably is so carried over in that excess phosgene increases the yield of acid chloride and eliminates the necessity of adding phosgene during the initial stages of the polymerization. It is desirable that the phosgene be present in an amount somewhat less than that actually required for total polymerization. This permits the avoidance of the formation of polyester blocks and polycarbonate blocks for the reasons described in columns 2 and 3 of U.S. Patent 4,194,038. It is not intended, however, to exclude from the present invention methods in which a high proportion such as 80 or 90 percent of the actually required phosgene (which may be 100% of that theoretically required without consideration of hydrolysis) is present in the organic solution at the beginning of polymerization. It appears that in interfacial polymerizations, unlike the solution polymerizations described in U.S. Patent 4,194,038, excessive haze (in the final polymer) does not occur merely because a large proportion (such as 90 or 100 percent) of the theoretically required phosgene is present with the acid chloride ahead of the dihydric alcohol.

Additional phosgene, if required in a poly(ester carbonate) synthesis, may be added after the polymerization has proceeded to a significant extent; and, preferably, phosgene is added after a substantial period of polymerization in an amount in excess of the stoichiometric amount to assure completion of the formation of the desired carbonate linkages.

As is conventional, other materials may be present during the polymerization, particularly phenolic chain regulator and terminator mateials such as tert-butylphenol. With routine experimentation, concentrations of such regulators can be determined for a particular combination and concentration of acid chloride and bisphenol to achieve a desired polymer size (as measured by reduced viscosity).

The polymerization reaction conditions are conventional for interfacial production of polyesters and polycarbonates and include sufficient agitation to produce the desired reaction between reactants in different phases. If a reaction results in phases difficult to separate, then greater agitation or other known methods should be tried to achieve clear phase separation. As is conventional, the temperatures and pressures of polymerization are not critical, with approximately atmospheric pressure and room temperature being convenient.

## Examples

### Starting Materials

The carboxylate acid salts used in the following examples were prepared by neutralization of the free acid in water and drying. The following procedure is typical.

A 4000 mL Erlenmeyer flask equipped with a magnetic spinbar was charged with 2000 mL 0.9919 M aqueous sodium hydroxide (1.9838 mol). The solution was stirred rapidly as 164.00 g (0.9872 mol) fibergrade terephthalic acid was added in portions. The solution was filtered, evaporated and dried in vacuo at 120°C overnight. The resulting white powder was passed through a 60-mesh stainless steel sieve and redried.

The pH of a 0.1581 g sample dissolved in approximately 10 mL distilled water was 9.4. Potentiometric titration of this solution required 15.21 mL of 0.0996 M aqueous hydrochloric acid indicating an equivalent weight of 104.4 g/carboxylate (theory requires 105.5 g/carboxylate).

The remaining raw materials were purchased except for 4,4'-benzophenonedicarboxylic acid and 2,6-naphthalenedicarboxylic acid, which were obtained by hydrolysis of the corresponding methyl esters.

### Example 1

A 500 mL flask equipped with mechanical stirrer, thermometer, phosgene dip tube, nitrogen inlet and dry ice condenser attached to a caustic scrubber was charged with 200 mL dichloromethane. The flask was immersed in an ice bath, the temperature was maintained at 2—4°C with stirring at approximately 200 rev/min while 33.66 g (0.34 mole) phosgene was introduced over 65 minutes. After 0.16 mL (2.0 mmol) pyridine was added, the temperature of the reaction mixture was raised to 25°C. A stirred slurry of 21.03 g (0.10 mol) disodium terephthalate and 0.51 g fluorenone as internal standard in 60 mL dichloromethane was added over 60 min. A moderate exotherm and steady evolution of carbon dioxide were noted. The reaction mixture was heated to 35°C and stirred an additional 90 min until the evolution of gas ceased.

A 1 mL aliquot of the slurry was removed and added to a mixture containing 20 mL methanol and 5 mL pyridine. After warming at 35°C for 15 min., the quenched aliquot was diluted to 100 mL with dichloromethane and analyzed by high performance liquid chromatography (HPLC). Comparison with standard mixtures of fluorenone and dimethyl terephthalate indicated 97.4% (+ or — 0.5%) conversion to terephthaloyl chloride (analyzed as dimethyl terephthalate).

Examples 2—6

Example 1 was repeated using 0.10 mol disodium terephthalate and the amounts of dichloromethane, moles of phosgene, millimoles of base, time and reaction temperature shown in Table 1. After the indicated time, the conversion was measured by esterifying an aliquot with methanol and measuring the amount of dimethyl terephthalate by high performance liquid chromatography (HPLC) as in Example 1. The conversions are shown in Table I.

Example 7

Example 1 was repeated using again 0.10 mol disodium terephthalate, 270 mL total dichloromethane, 0.32 mole phosgene and, instead of pyridine, 3.0 mmol triethylamine (TEA) with the reaction conducted at 35°C. Aliquots were withdrawn, esterified and analyzed as in Example 1. The conversions were as shown in Table I.

TABLE I

PYRIDINE AND 0.1 MOL DISODIUM TEREPHTHALATE

| Example (Temp.) | DCM (mL) | Phosgene (mol) | Pyridine (mmol) | Time (min) | Conversion (%) |
|---|---|---|---|---|---|
| 1 (35°C) | 250 | 0.34 | 2.0 | 90 | 97 |
| 2 (25°C) | 480 | 0.34 | 3.1 | 60 | 90 |
| | | | | 240 | 100 |
| 3 (25°C) | 480 | 0.31 | 1.0 | 5 | 41 |
| | | | | 60 | 60 |
| | | | | 240 | 81 |
| 4 (25°C) | 240 | 0.30 | 2.0 | 30 | 75 |
| | | | | 120 | 93 |
| | | | | 240 | 97 |
| 5 (35°C) | 250 | 0.32 | 1.0 | 240 | 94 |
| 6 (35°C) | 250 | 0.32 | 2.0 | 120 | 96 |
| 7 (35°C) | 270 | 0.32 | 3.0* | 120 | 5 |
| | | | | 240 | 11 |

* Triethylamine (mmol)

Example 8

A 1000-mL flask fitted with a mechanical stirrer, thermometer, phosgene diptube, nitrogen inlet and acetone-dry ice condenser attached to a caustic scrubber was charged with 400 mL dichloromethane. The flask was immersed in an ice bath, and the temperature was maintained at 2—4°C with stirring at approximately 200 rev/min while 25.20 g (254.9 mol) phosgene was introduced over 50 minutes. After 1.7 mL (21.0 mmol) pyridine was introduced, the temperature of the reaction mixture was raised to 25°C and the stirring rate was increased to 600 rev/min. A stirred slurry of 22.20 g (105.7 mmol) disodium terephthaltes in 100 mL dichloromethane was added over 60 minutes. A moderate exotherm and steady evolution of carbon dioxide were noted. The reaction mixture was stirred an additional 60 minutes before 18.18 g dimethyl adipate was added as an internal standard. At intervals indicated in Table II after the completion of disodium terephthalate addition, 5 mL aliquots of the reaction mixture were removed and added to a mixture containing 5 mL pyridine and 10 mL methanol. The quenched aliquots were analyzed by gas liquid partition chromatograph (GLPC) and compared with standard mixtures of dimethyl adipate and dimethyl terephthalate. The results are displayed in Table II.

Examples 9—13

Example 8 was repeated using the proportions of disodium terephthalate, phosgene and pyridine indicated in Table II. Quenched aliquots taken the indicated number of hours after all of the disodium terephthalate had been added were analyzed by GLPC, showing the yields indicated in Table II.

Examples 14—17

Example 8 was repeated using about 500 mL dichloromethane, the dipotassium or calcium salts of terephthalic acid and the quantities indicated in Table III, with the results indicated in Table III. In Example 15 dimethyl adipate was added to the aliquots rather than to the entire reaction mixture.

Examples 18—23

Example 8 was repeated using about 500 mL dichloromethane, the disodium salts of trans-1,4-cyclohexanedicarboxylic acid, isophthalic acid, 2,6-naphthalenedicarboxylic acid and 4,4′-benzophenonedicarboxylic acid, and the quantities indicated in Table IV, with the results indicated in Table IV.

Examples 24—30

Example 8 was repeated using about 500 mL dichloromethane and the quantities and tertiary nitrogen base indicated in Table V, with the results indicated in Table V. In Examples 24, 26 and 30 dimethyl adipate was added to the aliquots rather than to the entire reaction mixture. Examples 29 and 30 utilized a base with a pyridine nucleus, as is preferred. Example 28 utilized quinoline, which is less preferred, but still preferred over the bases having a nitrogen attached to three separate substituents as in Examples 24—27.

TABLE II

PYRIDINE AND DISODIUM TEREPHTHALATE
AT 25°C IN ABOUT 500mL DCM

| Example | Salt mmol | Phosgene mmol | Pyridine mmol | Time (hrs) | Conversion (%) |
|---|---|---|---|---|---|
| 8 | 105.7 | 254.9 | 21.0 | 1.1 | 92 |
| | | | | 2.1 | 96 |
| | | | | 3.1 | 98 |
| | | | | 19.5 | 99 |
| 9 | 111.6 | 234.1 | 4.5 | 0.2 | 56 |
| | | | | 0.8 | 81 |
| | | | | 1.7 | 83 |
| 10 | 108.1 | 237.6 | 10.5 | 1.2 | 94 |
| | | | | 2.1 | 95 |
| | | | | 4.0 | 95 |
| 11 | 106.0 | 232.4 | 21.1 | 0.5 | 96 |
| | | | | 0.8 | 97 |
| | | | | 1.5 | 97 |
| | | | | 3.0 | 97 |
| 12 | 101.3 | 304.9 | 30.0 | 1.1 | 91 |
| | | | | 2.2 | 97 |
| | | | | 4.1 | 98 |
| 13 | 109.6 | 242.2 | 42.0 | 1.2 | 88 |
| | | | | 2.1 | 94 |
| | | | | 3.1 | 95 |

TABLE III

PYRIDINE AND TEREPHTHALATE SALTS

| Example | Terephthalate mmol | Phosgene mmol | Pyridine mmol | Time (hrs) | Conversion (%) |
|---|---|---|---|---|---|
| 14 | K 99.7 | 299.4 | 21.0 | 1.1 | 98 |
| | | | | 2.0 | 99 |
| | | | | 3.0 | 100 |
| 15 | K 108.0 | 237.1 | 23.2 | 0.8 | 96 |
| | | | | 3.2 | 96 |
| 16 | Ca 107.1 | 236.2 | 21.5 | 2.5 | 16 |
| | | | | 4.5 | 19 |
| 17 | Ca 102.2 | 305.4 | 21.0 | 2.0 | 15 |
| | | | | 18.5 | 22 |

TABLE IV

PYRIDINE AND SODIUM SALTS

| Example | Sodium Salt* mmol | Phosgene mmol | Pyridine mmol | Time (hrs) | Conversion (%) |
|---|---|---|---|---|---|
| 18 | t–1, 4–C 111.2 | 245.5 | 22.3 | 1.5 | 88 |
| | | | | 3.0 | 90 |
| 19 | t–1, 4–C 101.6 | 305.6 | 21.0 | 1.1 | 86 |
| | | | | 2.0 | 87 |
| | | | | 3.7 | 87 |
| 20 | IP 102.3 | 309.3 | 21.0 | 1.2 | 90 |
| | | | | 2.1 | 92 |
| | | | | 3.7 | 93 |
| 21 | IP 100.8 | 308.3 | 21.0 | 1.1 | 89 |
| | | | | 2.0 | 89 |
| | | | | 3.7 | 91 |
| 22 | 2,6–N 103.8 | 313.4 | 30.0 | 1.2 | 97 |
| | | | | 2.7 | 95 |
| 23 | 4,4′–BP 101.9 | 306.7 | 30.0 | 1.2 | 93 |
| | | | | 2.8 | 93 |

* t–1,4–C is the disodium salt of trans-1,4-cyclohexane dicarboxylic acid

IP is the disodium salt of isophthalic acid

2,6–N is the disodium salt of 2,6-naphthalenedicarboxylic acid

4,4′–BP is the disodium salt of 4,4′-benzophenonedicarboxylic acid

TABLE V

OTHER BASES AND DISODIUM TEREPHTHALATE

| Example | Salt (mmol) | Phosgene (mmol) | Base* | mmol | Time (hrs) | Conversion (%) |
|---|---|---|---|---|---|---|
| 24 | 109.5 | 240.4 | TEA | 21.8 | 1.8<br>4.3 | 8<br>21 |
| 25 | 102.6 | 308.6 | TEA | 21.5 | 1.1<br>3.0 | 5<br>14 |
| 26 | 104.0 | 242.8 | N,N—DMA | 22.2 | 0.8<br>3.0 | 9<br>27 |
| 27 | 101.0 | 300.6 | N,N—DMA | 21.0 | 1.3<br>3.1<br>18.5 | 8<br>18<br>72 |
| 28 | 100.8 | 301.5 | Q | 21.0 | 1.3<br>3.5 | 25<br>46 |
| 29 | 104.0 | 311.8 | 4—P | 21.0 | 1.1<br>3.0<br>19.0 | 93<br>95<br>93 |
| 30 | 109.1 | 248.4 | 4—DMAP | 22.5 | 1.5<br>3.5 | 65<br>88 |

* TEA is triethylamine

N,N—DMA is N,N-dimethylaniline

Q is quinoline

4—P is 4-picoline or 4-methylpyridine

4—DMAP is 4-dimethylaminopyridine

Example 31

This example illustrates the use of the product acid chloride solution of the present invention for interfacial synthesis.

A 500 mL flask equipped with mechanical stirrer, thermometer, phosgene dip tube, nitrogen inlet and dry ice condenser attached to a caustic scrubber was charged with 200 mL dichloromethane. The flask was immersed in an ice bath, the temperature was maintained at 2—4°C with stirring at approximately 200 rev/min while 31.6 g (319 mmol) phosgene was introduced over 65 minutes. After 0.16 mL (2 mmol) pyridine was added, the temperature of the reaction mixture was raised to 25°C. A stirred slurry of 21.53 g (103 mmol) disodium terephthalate in 60 mL dichloromethane was added over 60 min. A moderate exotherm and steady evolution of carbon dioxide were noted. The reaction mixture was heated to 35°C and stirred an additional 90 min until the evolution of gas ceased.

The slurry was cooled in an ice bath to 2—4°C and filtered under slight nitrogen pressure through a medium sintered glass fritte. The reaction flask and filtercake were washed with three 70 mL portions of dichloromethane.

The combined solution of about 470 mL containing about 103 mmol terephthaloyl chloride and 114 mmol phosgene was added with a 30 mL dichloromethane solution containing 0.36 g (2.40 mmol) tertbutylphenol to a well stirred aqueous solution of sodium bisphenolate (205 mmol in about 298 mL) and triethylamine (0.65 mmol). The reaction mixture turned yellow momentarily, but after 2—3 minutes became milky. A slight (5—6°C) temperature increase was noted.

After 20 min the pH was 2—3 and an aliquot was removed for analysis. Aqueous sodium hydroxide (140 mL containing 192 mmol) was added to bring the pH back to above 11 and the reactor was swept with phosgene gas until the pH returned to the 5—6 range (5—6 minutes at about 1 g phosgene/min). The mixture increased in viscosity and, after 40 minutes and again after 60 minutes,

stirring was momentarily discontinued and the phases separated immediately into a clear aqueous phase and a thick, milky organic phase. Aliquots were taken from the organic phase each time.

Then 40 mL of aqueous sodium hydroxide (55 mmol) was added, bringing the pH to above 11, and the mixture was stirred for 30 min more. Again the phases separated immediately and all of the organic layer was taken as the fourth sample.

The first three samples of organic layer were worked up by precipitation in methanol, dissolution in dichloromethane, water extraction until a negative chloride ion test was obtained, filtration, reprecipitation in isopropanol, and vacuum drying (120°C, 1 torr or 133 Pa for 24 h). All separations were quick and sharp with some slight amount of insoluble material present.

The final (fourth) sample was worked up by separating the organic phase from the water phase, diluting it with more dichloromethane, precipitation of the polymer by addition to isopropanol (blender) and washing with isopropanol. The product was then redissolved in dichloromethane and extracted with distilled water until a negative chloride ion test was obtained. It was finally filtered, precipitated by adding to isopropanol, and vacuum dried (120°C, 1 torr or 133 kPa for 24 hr). Some difficulty was initially encountered with the work-up due to the high viscosity of the final sample but this was overcome by a 4—6 fold dilution with dichloromethane.

Reduced viscosities of the four samples were measured in phenol-tetrachloroethane and were 0.29, 1.61, 1.58 dL/g for the four samples. Infrared analysis of the fourth sample shows a 1.94:1 ratio of bisphenol A derived residues to terephthalate residues. Differential scanning calorimetry (DSC) of the fourth sample indicated a glass transition (Tg) at 192°C and thermal gravimetric analysis (TGA) showed major decomposition above 400°C in a single step, with less than 1% weight loss at 300°C and 2.5% weight loss at 400°C.

Discs of 1/8 inch thickness by 1—1/4 inch diameter (3.18 mm by 31.75 mm) molded from the fourth sample at 310°C exhibited an 18.6 yellowness index, 80.1% transmittance value and 10.9 haze index according to ASTM D-1925 as used in U.S. Patent 4,156,069.

### Example 32

Example 31 was repeated with the following proportions used in preparation of the terephthaloyl chloride solution:

200 mL dichloromethane
2 mmol pyridine
315 mmol phosgene
105 mmol disodium terephthalate (added over 30 rather than 60 minutes)

After the by-product sodium chloride was filtered out, about 470 mL of solution was reacted as in Example 31. Three aliquots and a fourth, final sample were taken during reaction as in Example 31 and worked up as in Example 31. The reduced viscosities for the four samples were 0.27, 1.40, 1.38 and 1.92 dL/g, the ratio of bisphenol A derived residues to terephalate residues for the fourth sample was 1.95 by infrared, the Tg was 195°C by DSC and the weight loss by TGA was under 1% at 300°C and 1.3% at 400°C, with decomposition in a single step above 400°C. Discs molded at 310°C showed a 13.2 yellowness index, 83.5% transmittance and a 12.0 haze index by ASTM D-1925.

It will be appreciated that lower molecular weights, as for example in the 0.5 to 1.0 dL/g reduced viscosity range, can be obtained by adding more t-butylphenol regulator, with the amount determinable by routine experimentation.

### Example 33

Example 31 is repeated using, in place of disodium terephthalate, about 100 mmol of the following salts:

A) dipotassium terephthalate
B) calcium terephthalate
C) disodium isophthalate
D) disodium 4,4′-benzophenonedicarboxylate
E) disodium 2,6-naphthalenedicarboxylate
F) disodium 1,4-cyclohexanedicarboxylate

The times necessary for relatively complete conversion can be determined by routine experimentation. It can be seen from Examples 14—23, above, that the corresponding acid chlorides will be formed. Polymerization is then conducted interfacially to produce the corresponding poly(ester carbonate).

### Example 34

Example 31 is repeated through the end of carbon dioxide evolution at 35°C. The reaction mixture is then heated to near 40°C and both phosgene and some dichloromethane and removed by evaporation. The remaining slurry is then filtered as in Example 31 and the filtercake washed as in Example 31, but with sufficient dichloromethane used to bring the combined filtrate solution up to 500 mL.

The resultant 500 mL solution containing about 100 mmol terephthaloyl chloride is reacted interfacially with an aqueous sodium bisphenolate solution (about 100 mmol in about 150 mL) in the presence of about 0.35 mmol triethylamine. Agitation is stopped momentarily every twenty or so minutes and aliquots of organic layer are taken and analyzed for viscosity of the organic phase. Reaction is continued until polymer of the desired molecular weight is formed. If the molecular weight is higher than desired, then the experiment is repeated with various amounts of a regulator such as tert-butylphenol until the desired molecular weight is achieved.

Example 35

Example 34 is repeated, using, in place of disodium terephthalate, 100 mmol of the following salts:

A) dipotassium terephthalate
B) calcium terephthalate
C) disodium isophthalate
D) disodium terephthalate (50 mmol) and disodium isophthalate (50 mmol)
E) disodium 4,4'-benzophenonedicarboxylate
F) disodium 2,6-naphthalenedicarboxylate
G) disodium 1,4-cyclohexanedicarboxylate

Once the acid chlorides are formed as in Examples 14—23, after reaction times determinable with routine experimentation, then removal of phosgene and of solids, and then polymerization is conducted as in Example 34 to produce the corresponding polyester.

Example 36

A solution of 0.05 mol of terephthaloyl chloride (TPC) in 250 mL dichloromethane was filtered through a fine sintered glass funnel, and about 0.055 mol condensed phosgene was added. Pyridine was then added in some runs (0.5 mmol in Runs C and J; 1.5 mmol in Runs D and E; 2.5 mmol in Run F; 3.5 mmol in Run G; 5.0 mmol in Runs H and I) to simulate pyridine carried over from TPC synthesis.

A 146 mL aqueous solution containing sodium hydroxide (0.20 mol), 0.10 mol of bisphenol-A and triethylamine (TEA) (in amounts indicated in Table I) were charged to a three-necked flask equipped with mechanical stirrer operating at a rate of about 575 rev/min. A solution of 0.18 g (1.2 mmol) tert-butylphenol in 15 mL dichloromethane and the above TPC-containing solution were charged simultaneously to the above aqueous bisphenol-A/triethylamine solution. A slight temperature rise (about 5°C) was observed from room temperature and, in runs with pyridine present, an orange color appeared for about 2—3 minutes. Thereafter the reaction mixture turned milky and increased in viscosity.

After twenty minutes the pH decreased to 3—6. Agitation was stopped momentarily, and the reaction mixture separated into a cloudy aqueous phase and a clear organic phase (containing dissolved polymer with low reduced viscosities in the range of 0.11 to 0.46 dL/g indicative of short oligomers). Stirring was resumed, 0.1 mol of sodium hydroxide was added and phosgene gas was passed over the reaction mixture until the pH decreased back to 7—8 range (aliquots of organic layer being taken in most cases after 20 and 40 additional minutes of total reaction time — other sampling times are indicated in Table VI). More aqueous sodium hydroxide (27 mmol) was added to bring the pH up to the 10 to 11 range and the mixture was stirred for an additional 30 minutes.

The various samples of organic layer were precipitated in methanol, filtered, redissolved in dichloromethane and repeatedly washed with distilled water until the wash water tested negatively for chloride ion. The solution was filtered through a sintered glass funnel, precipitated by addition to isopropanol, filtered and vacuum dried at 110°C and 133 Pa (1 torr).

In addition, with pyridine contents of 3 mole % or less (by moles of diacid chloride) the reaction mixtures separated quickly into two phases, and the subsequent work-up was facilitated, with very little sludge formation. Reduced viscosities of each sample (in phenol-tetrachloroethane) were measured and are reproduced in Table VI.

TABLE VI

| Run | Pyridine mmol | TEA mmol | Polymerization time (min) | Reduced Viscosity |
|---|---|---|---|---|
| A | 0 | 0.32 | 23 | 0.36 |
| | | | 63 | 0.85 |
| | | | 142* | 0.90 |
| B | 0 | 0.37 | 20 | 0.46 |
| | | | 40 | 1.75 |
| | | | 60 | 1.69 |
| | | | 90 | 1.77 |
| C | 0.5 | 0.32 | 20 | 0.25 |
| | | | 40 | 1.45 |
| | | | 60 | 1.49 |
| | | | 90 | 1.54 |
| D | 1.5 | 0.32 | 21 | 0.19 |
| | | | 60 | 0.60 |
| | | | 120* | 0.58 |
| E | 1.5 | 0.32 | 20 | 0.20 |
| | | | 40 | 0.56 |
| | | | 60 | 0.59 |
| | | | 90 | 0.60 |
| F | 2.5 | 0.32 | 20 | 0.16 |
| | | | 40 | 0.32 |
| | | | 60 | 0.45 |
| | | | 90 | 0.50 |
| G | 3.5 | 0.32 | 20 | 0.16 |
| | | | 40 | 0.31 |
| | | | 60 | 0.31 |
| | | | 90 | 0.43 |
| H | 5.0 | 0.32 | 30 | 0.15 |
| | | | 90 | 0.26 |
| | | | 160 | 0.26 |
| I | 5.0 | 0 | 20 | 0.13 |
| | | | 30 | 0.20 |
| | | | 55 | 0.24 |
| J | 0.5 | 0 | 20 | 0.28 |
| | | | 40 | 0.29 |
| | | | 60 | 0.16 |
| | | | 90 | 0.30 |

*Times greater than 90 minutes in Runs A, D and H resulted from resuming stirring after 60 minutes for varying periods before raising the pH to 11. In each case, base was added after the penultimate sampling, and stirring was resumed for about 30 minutes before the final sample was taken. In some cases, further reaction only lowered the reduced viscosity suggesting that degradation was occurring rather than further polymerization.

The results indicate that low pyridine content in Runs C—E (0.5 to 1.5 mmol or 1—3% by moles of TPC) did not interfere with polymerization to an excessive degree, since reduced viscosities of about 0.50 dL/g were still obtainable. Runs F—H, in which the pyridine content was higher (2.5 to 5.0 mmol or 5—10% by moles of TPC), showed insufficient polymer length or molecular weight being achieved as evidenced by reduced viscosities no greater than 0.50 and generally about 0.4 dL/g or less. It thus appears that, for pyridine, the purchased terephthalic acid chloride and the regulator used in this

Example, tolerable levels are up to about 3%, by moles of TPC, with up to about 2% being preferred. These levels correspond to 1.5% and 1.0%, by moles of carbonyl chloride.

It should be appreciated, however, that Example 31 had about 102 mmol terephthaloyl chloride of high reactivity and 2 mmol pyridine (2% by moles of diacid chloride or 1% by moles of carbonyl chloride) and, with comparable regulator levels and reaction conditions, produced polymer of 1.85 dL/g in 90 minutes. This greater value compared to Runs C, D and E of this Example 36 (with 1, 3 and 3% pyridine by moles of diacid chloride) suggests that more pyridine could be tolerated in the polymerization of Example 31 or 32 because of the better acid chloride quality. It is on this basis that the preferred range of about 0.1 to 10% and more preferred range of about 0.2 to 5% of tertiary nitrogen compound with a pyridine nucleus, by moles of carboxylate or carbonyl chloride, have been chosen rather than the lower ranges suggested by Table VI.

Example 37

This example illustrates the use of the product solution of the present acid chloride invention to produce poly(ester carbonate) by a solution process.

A 100 mL flask fitted with a mechanical stirrer, thermometer, phosgene diptube, nitrogen inlet and dry ice condenser attached to a caustic scrubber was charged with 400 mL dichloromethane. The flask was immersed in an ice bath, and the temperature was maintained at 2—4°C with stirring at approximately 200 rev/min while 30.74 g (310.7 mmol) phosgene was introduced over 60 minutes. After 2.40 mL (29.7 mmol) pyridine was added, the temperature of the reaction mixture was raised to 25°C, and the stirring rate was increased to 600 rev/min. A stirred slurry of 21.75 g (103.5 mmol) disodium terephthalate in 100 mL dichloromethane was added over 60 minutes. A moderate exotherm and steady evolution of carbon dioxide were noted. The reaction mixture was stirred an additional 2 hours at 25°C and then filtered under slight nitrogen pressure through a medium sintered glass fritte. The clear, light yellow filtrate was reduced to about 150 ml on a rotary evaporator.

A 1000 mL flask fitted with a mechanical stirrer, thermometer, addition funnel, nitrogen inlet and dry ice condenser attached to a caustic scrubber was charged with 47.30 g (207.2 mmol) bisphenol A, 60.0 mL (741.8 mmol) pyridine and 400 mL dichloromethane. The temperature was maintained at 25°C with a stirring rate of 500 rev/min throughout subsequent reaction steps. The terephthaloyl chloride solution prepared above was added over 50 minutes followed by a 20 minute holding period.

In order to control the molecular weight achieved in the final condensation step, 0.65 g (4.3 mmol) t-butylphenol was added to the mixture of bisphenol A terephthalate oligomers. The final portion of phosgene was introduced over 35 minutes. The viscous reaction mixture was stirred an additional 45 minutes and then quenched by the careful addition of 50 mL methanol.

A 250-mL portion of water was added and the resulting heterogeneous mixture was emulsified in a flask equipped with a stopcock drain and a mechanical stirrer operating at approximately 700 rev/min. Precipitation was carried out in a stainless steel blender by addition of the emulsion over 15—20 minutes to 2000 mL isopropanol which was initially heated to 60—65°C and rapidly stirred. The solids were filtered and washed sequentially with 1000 mL portions each of isopropanol, and water initially heated to 90—100°C overnight to give 61.83 g (96.4% of theory) poly(ester-carbonate).

Analyses of composition employing standard infrared and nuclear magnetic resonance (NMR) techniques each indicated a terephthalate to bisphenol A ratio of 0.51. The reduced viscosity of a 0.5% (w/v) copolymer solution in a mixture of phenol and sym-tetrachloroethane (60:40 by weight) was 1.03 dL/g. Differential scanning calorimetry (DSC) and thermal gravimetric analysis (TGA) revealed that there was a well defined glass transition at 185°C and no significant weight loss below 380°C in an argon atmosphere. A 3.2 mm plaque compression molded at 250°C (315°C) exhibited a transmission haze value of 4.3 (5.3), a yellowness index of 5.8 (10.5) and a percent transmittance of 90.0 (87.2). The values in parenthesis are for 315°C molding.

Example 38

Example 37 was repeated using the disodium salt of isophthalic acid and the following quantities:

400 mL dichloromethane
30.61 g (309.4 mmol) phosgene
2.40 mL (29.7 mmol) pyridine
20.78 g (98.9 mmol) disodium isophthalate

The product solution containing isophthaloyl chloride was evaporated to 100 mL in the rotary evaporator. The charge to the polymerization flasks was:

45.20 g (198.0 mmol) bisphenol A
60.0 mL (741.8 mmol) pyridine
400 mL dichloromethane
Isophthaloyl chloride solution (over 40 minutes)
0.61 g (4.1 mmol) *t*-butylphenol

After washing and precipitation as in Example 33 and vacuum drying, the product was 57.80 g (94.3% of theory) poly(ester carbonate).

Analyses of composition employing standard infrared and nuclear magnetic resonance (NMR)

techniques indicated isophthalate to bisphenol A ratios of 0.48 and 0.54 respectively. The reduced viscosity of a 0.5% (w/v) copolymer solution in a mixture of phenol and sym-tetrachloroethane (60:40 by weight) was 0.70 dL/g. Differential scanning calorimetry (DSC) and thermal gravimetric analysis (TGA) revealed that there was a well defined glass transition at 170°C and no significant weight loss below 390°C in argon atmosphere. A 3.2 mm plaque compression molded at 250°C (315°C) exhibited a transmission haze value of 12.4 (6.6, a yellowness index of 5.5 (7.6) and a percent transmittance of 90.6 (87.2).

## Claims

1. A method of preparing an aromatic or cycloaliphatic dicarboxylic acid chloride which comprises reacting an alkali metal or alkaline earth metal salt of an aromatic or cycloaliphatic dicarboxylic acid in an inert organic solvent with phosgene in the presence of at least one tertiary nitrogen compound with a pyridine nucleus in a molar amount of 0.1% to 25%, by moles of carboxylate to form the corresponding aromatic or cycloaliphatic dicarboxylic acid chloride dissolved in the organic solvent, carbon dioxide and a solid alkali metal or alkaline earth metal chloride salt.

2. The method of claim 1 wherein the alkali metal or alkaline earth metal of an aromatic or cycloaliphatic dicarboxylic acid is an alkali metal salt of an aromatic dicarboxylic acid.

3. The method of claim 2 wherein the alkali metal salt of an aromatic dicarboxylic acid is the disodium or dipotassium salt of terephthalic or isophthalic acid.

4. The method of any previous claim wherein the at least one tertiary nitrogen compound with a pyridine nucleus is present in a molar amount of 0.2% to 5%, by moles of carboxylate.

5. The method of any previous claim wherein the at least one tertiary nitrogen compound is pyridine.

6. The method of any previous claim further comprising separating the solid alkali metal or alkaline earth metal chloride salt from the organic solution.

7. A process for the production of a polyester which comprises:

a) forming an aromatic or cycloaliphatic dicarboxylic acid chloride dissolved in the organic solvent, carbon dioxide and an alkali metal or alkaline earth metal chloride salt suspended in the organic solution by the method of any of claims 1 to 6;

b) removing any unreacted phosgene from the organic solution; and

c) mixing the organic solution with an aqueous solution of an alkali metal or alkaline earth metal salt of a dihydric aromatic alcohol under sufficient agitation and with sufficient tertiary nitrogen polymerization catalyst present to form a polyester polymer.

8. A process for the production of a poly(ester carbonate) which comprises:

a) forming an aromatic or cycloaliphatic dicarboxylic acid chloride dissolved in an inert organic solvent, carbon dioxide and an alkali metal or alkaline earth metal chloride salt suspended in the organic solution by reacting an alkali metal or alkaline earth metal salt of an aromatic or cycloaliphatic dicarboxylic acid in an inert organic solvent with phosgene in the presence of at least one tertiary nitrogen compound with a pyridine nucleus in a molar amount of 0.1% to 25%, by moles of carboxylate;

b) mixing the organic solution with an aqueous solution of an alkali metal or alkaline earth metal salt of a dihydric aromatic alcohol under sufficient agitation and with sufficient phosgene and tertiary nitrogen polymerization catalyst present to form a poly(ester carbonate) polymer.

9. The process of claim 8 wherein said at least one tertiary nitrogen compound is pyridine, present in the amount between 0.1 and 10%, by moles of carboxylate.

10. The process of claim 9 wherein pyridine is present in an amount between 0.2% and 5%, by moles of carboxylate.

11. The process of claim 8 or 9 or 10 wherein the alkali metal or alkaline earth metal salt of an aromatic or cycloaliphatic dicarboxylic acid is an alkali metal salt of an aromatic dicarboxylic acid.

12. The process of any of claims 8—11 wherein the alkali metal salt of an aromatic dicarboxylic acid is the disodium or dipotassium salt of terephthalic or isophthalic acid.

13. The process of any of claims 8—12 wherein the organic solution is mixed with the aqueous solution in the presence of a catalytic amount of a tertiary amine polymerization catalyst, with phosgene also being present, to form the poly(ester carbonate), and the process further comprises:

(c) separating aqueous and organic phases after the poly(ester carbonate) is formed,

(d) washing the organic phase substantially free of halide, and

(e) recovering the poly(ester carbonate) from the washed organic phase.

14. The process of claim 13 wherein the phosgene is substantially all present in the organic solution before mixing.

15. The process of any of claims 7—14 wherein the aromatic dihydric alcohol is bisphenol A.

16. The process of any of claims 7—15 wherein the solid alkali metal or alkaline earth metal chloride salt is separated from the organic solution before mixing the organic solution with the aqueous solution.

**Patentansprüche**

1. Verfahren zum Erzeugen eines aromatischen oder cycloaliphatischen Dicarbonsäurechlorids, dadurch gekennzeichnet, daß ein Alkalimetallsalz oder Erdalkalimetallsalz einer aromotischen oder cycloaliphatischen Dicarbonsäre in einem inerten organischen Lösungsmittel in Gegenwart mindestens einer tertiären Stickstoffverbindung mit einem Pyridinkern in einer auf das Carboxylat bezogenen Menge von 0,1 bis 25 Molprozent mit Phosgen umgesetzt und dadurch das in dem organischen Lösungsmittel gelöste, entsprechende aromatische oder cycloaliphatische Dicarbonsäurechlorid, sowie Kohlendioxid und ein festes Alkalimetallchloridsalz oder Erdalkalimetallchloridsalz gebildet werden.

2. Verfahren nach Anspruch 1, dadurch gekennzeichnet, daß das Alkalimetallsalz oder Erdalkalimetallsalz einer aromatischen oder cycloaliphatischen Dicarbonsäure ein Alkalimetallsalz einer aromatischen Dicarbonsäure ist.

3. Verfahren nach Anspruch 2, dadurch gekennzeichnet, daß das Alkalimetallsalz einer aromatischen Dicarbonsäure das Dinatrium- oder Dikaliumsalz der Terephthalsäure oder der Isophtalsäure ist.

4. Verfahren nach einem der vorhergehenden Ansprüche, dadurch gekennzeichnet, daß die mindestens eine tertiäre Stickstoffverbindung mit einem Pyridinkern in einer auf das Carboxylat bezogenen Menge von mindestens 0,2 bis 5 Molprozent vorhanden ist.

5. Verfahren nach einem der vorhergehenden Ansprüche, dadurch gekennzeichnet, daß die mindestens eine tertiäre Stickstoffverbindung Pyridin ist.

6. Verfahren nach einem der vorhergehenden Ansprüche dadurch gekennzeichnet, daß das feste Alkalimetall oder Erdalkalimetallchloridsalz von der organischen Lösung abgetrennt wird.

7. Verfahren zum Erzeugen eines Polyesters, dadurch gekennzeichnet, daß

a) nach dem Verfahren nach einem der Ansprüche 1 bis 6 ein in dem organischen Lösungsmittel gelöstes, aromatisches oder cycloaliphatisches Dicarbonsäurechlorid, Kohlendioxid und ein in der organischen Lösung suspendiertes Alkalimetall oder Erdalkalimetallchloridsalz erzeugt werden.

b) nicht umgesetztes Phosgen von der organischen Lösung abgetrennt wird und

c) die organische Lösung mit einer wässrigen Lösung eines Alkalimetall- oder Erdalkalimetallsalzes eines zweiwertigen aromatischen Alkohols vermischt und dabei so viel gerührt wird und eine solche Menge einer tertiären Stickstoffverbindung als Polymerisationskatalysators anwesend ist, daß ein polymeres Polyester gebildet wird.

8. Verfahren zum Erzeugen eines Poly(estercarbonats), dadurch gekennzeichnet, daß

a) durch Umsetzen eines Alkalimetall- oder Erdalkalimetallsalzes einer aromatischen oder cycloaliphatischen Dicarbonsäure in Gegenwart mindestens einer tertiären Stickstoffverbindung mit einem Pyridinkern in einer auf das Darboxylat bezogenen Menge von 0,1 bis 25 Molprozent mit Phosgen ein in einem inwerten organischen Lösungsmittel gelöstes, aromatisches oder cycloaliphatisches Dicarbonsäurechlorid, Kohlendioxid und ein in der organischen Lösung suspendiertes Alkalimetall- oder Erdalkalimetallchloridsalz erzeugt werden,

b) die organische Lösung mit einer wässrigen Lösung eines Alkalimetall- oder Erdalkalimetallsalzes eines zweiwertigen aromatischen Alkohols vermischt und dabei so viel gerührt wird und eine solche Menge einer tertiären Stickstoffverbindung als Polymerisationskatalysators anwesend ist, daß ein polymeres Poly(estercarbonat) erzeugt wird.

9. Verfahren nach Anspruch 8, dadurch gekennzeichnet, daß die mindestens eine tertiäre Stickstoffverbindung aus Pyridin besteht und in einer auf das Carboxylat bezogenen Menge von 0,1 bis 10 Molprozent vorhanden ist.

10. Verfahren nach Anspruch 9, dadurch gekennzeichnet, daß das Pyridin in einer auf das Carboxylat bezogenen Menge von 0,2 bis 5 Molprozent vorhanden ist.

11. Verfahren nach Anspruch 8 oder 9 oder 10, dadurch gekennzeichnet, daß das Alkalimetallsalz oder Erdalkalimetallsalz einer aromatischen oder cycloaliphatischen Dicarbonsäure ein Alkalimetallsalz einer aromatischen Dicarbonsäure ist.

12. Verfahren nach einem der Ansprüche 8 bis 11, dadurch gekennzeichnet, daß das Alkalimetallsalz einer aromatischen Dicarbonsäure das Dinatrium- oder Dikaliumsalz der Terephthalsäure oder der Isophtalsäure ist.

13. Verfahren nach einem der Ansprüche 8 bis 12, dadurch gekennzeichnet, daß zum Erzeugen des Poly(estercarbonats) die organische Lösung in mit der wässrigen Lösung in Gegenwart von Phosgen und in Gegenwart einer katalytisch wirksamen Menge eines tertiären Amins als Polymerisationskatalysator vermischt wird und daß ferner

(c) nach der Bildung des Poly)estercarbonats) die wässrige und die organische Phase voneinander getrennt werden,

(d) die organische Phase durch Waschen im wesentlichen von Halogenid befreit wird und

(e) das Poly(estercarbonat) aus der gewaschenen organischen Phase gewonnen wird.

14. Verfahren nach Anspruch 13, dadurch gekennzeichnet, daß das Phosgen vor dem Vermischen im wesentlichen vollständig in der organischen Lösung enthalten ist.

15. Verfahren nach einem der Ansprüche 7 bis 14, dadurch gekennzeichnet, daß der zweiwertige aromatische Alkohol Bisphenol A ist.

16. Verfahren nach einem der Ansprüche 7 bis 15, dadurch gekennzeichnet, daß das feste Alkalimetall- oder Erdalkalimetallchloridsalz von der organischen Lösung abgetrennt wird, bevor diese mit der wässrigen Lösung vermischt wird.

**Revendications**

1. Procédé de préparation d'un chlorure d'acide dicarboxylique aromatique ou cycloaliphatique qui consiste à faire réagir un sel de métal alcalin ou un sel de métal alcalino-terreux d'un acide dicarboxylique aromatique ou cycloaliphatique dans un solvant organique inerte avec du phosgène en présence d'au moins un composé azoté tertiaire ayant un noyau pyridine en une quantité molaire de 0,1% à 25% par mole de carboxylate pour former le chlorure d'acide carboxylique aromatique ou cycloaliphatique correspondant dissous dans le solvant organique, du dioxyde de carbone et un sel de chlorure de métal alcalin ou de métal alcalino-terreux à l'état solide.

2. Procédé selon la revendication 1 dans lequel le sel de l'acide dicarboxylique aromatique ou cycloaliphatique est un sel de métal alcalin d'un acide dicarboxylique aromatique.

3. Procédé selon la revendication 2 dans lequel le sel de métal alcalin d'un acide dicarboxylique aromatique est le sel disodique ou dipotassique de l'acide téréphtalique ou de l'acide isophtalique.

4. Procédé selon l'une quelconque des revendications précédentes dans lequel le (ou les) composé(s) azoté(s) tertiaire(s) ayant un noyau pyridine est (sont) présent(s) en une quantité molaire de 0,2% à 5% par mole de carboxylate.

5. Procédé selon l'une quelconque des revendications précédentes dans lequel le composé azoté tertiaire est la pyridine.

6. Procédé selon l'une quelconque des revendications précédentes comprenant en outre la séparation à l'état solide du chlorure de métal alcalin ou alcalino-terreux de la solution organique.

7. Procédé pour la production d'un polyester qui consiste:

a) à former un chlorure d'acide dicarboxylique aromatique ou cycloaliphatique dissous dans le solvant organique, du dioxyde de carbone et un chlorure de métal alcalin ou alcalino-terreux en suspension dans la solution organique selon le procédé de l'une quelconque des revendications 1 à 6;

b) à éliminer de la solution organique tout le phosgène qui n'a pas réagi;

c) à mélanger la solution organique avec une solution aqueuse d'un sel de métal alcalin ou alcalino-terreux d'un diol aromatique sous agitation suffisante et avec suffisamment de catalyseur de polymérisation azoté tertiaire pour former un polymère de polyester.

8. Procédé pour la production d'un poly(ester carbonate) qui consiste:

a) à former un chlorure d'acide dicarboxylique aromatique ou cycloaliphatique dissous dans un solvant organique inerte, du dioxyde de carbone et un chlorure de métal alcalin ou alcalino-terreux en suspension dans la solution organique en faisant réagir un sel de métal alcalin ou alcalino-terreux d'un acide dicarboxylique aromatique ou cycloaliphatique dans un solvant organique inerte avec du phosgène en présence d'au moins un composé azoté tertiaire ayant un noyau pyridine en une quantité molaire de 0,1% à 25%, par mole de carboxylate;

b) à mélanger la solution organique avec une solution aqueuse d'un sel de métal alcalin ou alcalino-terreux d'un diol aromatique sous agitation suffisante et avec suffisamment de phosgène et de catalyseur de polymérisation azoté tertiaire pour former un polymère de poly(ester carbonate).

9. Procédé selon la revendication 8 dans lequel le (au moins un) composé azoté tertiaire est la pyridine présente dans une quantité comprise entre 0,1 et 10% par mole de carboxylate.

10. Procédé selon la revendication 9 dans lequel la pyridine est présente en quantité comprise entre 0,2% et 5% par mole de carboxylate.

11. Procédé selon la revendication 8 ou 9 ou 10 dans lequel le sel métallique de l'acide dicarboxylique aromatique ou cycloaliphatique est un sel de métal alcalin d'un acide dicarboxylique aromatique.

12. Procédé selon l'une quelconque des revendications 8 à 11 dans lequel le sel de métal alcalin d'un acide dicarboxylique aromatique est le sel disodique ou dipotassique de l'acide téréphtalique ou isophtalique.

13. Procédé selon l'une quelconque des revendications 8 à 12 dans lequel la solution organique est mélangée avec la solution aqueuse en présence d'une quantité catalytique d'une amine tertiaire, comme catalyseur de polymérisation, du phosgène étant également présent, pour former le poly(ester carbonate) ledit procédé consistant, en outre:

c) à séparer les phases aqueuse et organique après formation du poly(ester carbonate),

d) à laver la phase organique pour en éliminer les halogénures,

e) à récupérer le poly(ester carbonate) de la phase organique lavée.

14. Procédé selon la revendication 13 dans lequel le phosgène est sensiblement présent en totalité dans la solution organique avant exécution du mélange.

15. Procédé selon l'une quelconque des revendications 7 à 14 dans lequel le diol aromatique est le bisphénol-A.

16. Procédé selon l'une quelconque des revendications 7 à 15 dans lequel le chlorure de métal alcalin ou alcalino-terreux, à l'état solide, est separé de la solution organique avant le mélange de cette dernière avec la solution aqueuse.